# EUROPÄISCHE PATENTSCHRIFT

(11) **EP 2 588 849 B1**
(45) Veröffentlichungstag und Bekanntmachung des Hinweises auf die Patenterteilung: **07.05.2014**
(21) Anmeldenummer: 10730149.1
(22) Anmeldetag: 01.07.2010
(51) Int. Cl.: G01N 27/28, G01N 33/18

(54) **WASSERANALYSE-SENSORKARTUSCHE MIT TRANSPORTBEHÄLTER**
WATER ANALYSIS SENSOR CARTRIDGE WITH TRANSPORT CONTAINER
CARTOUCHE DE CAPTEUR D'ANALYSE DE L'EAU COMPORTANT UN CONTENANT DE TRANSPORT

(43) Veröffentlichungstag der Anmeldung: 08.05.2013
(73) Patentinhaber: Hach Lange GmbH, 14163 Berlin (DE)
(72) Erfinder: LEYER, Axel, 41199 Mönchengladbach (DE); HEIDEMANNS, Lothar, 44575 Korschenbroich (DE); JONAK, Andreas, 40670 Meerbusch (DE); HAHN, Markus, 47906 Kempen (DE); RUDDE, Heinz, 41836 Hückelhoven (DE); RIEGER, Claudia, 40227 Düsseldorf (DE); STELLMACH-HANULOK, Aurelia, 42489 Wülfrath (DE); GOLITZ, Andreas, 47445 Moers (DE); KUSSMANN, Michael, 40476 Düsseldorf (DE)
(74) Vertreter: Patentanwälte ter Smitten Eberlein Rütten Partnerschaftsgesellschaft
(86) Internationale Anmeldenummer: PCT/EP2010/059332
(87) Internationale Veröffentlichungsnummer: WO 2012/000552

(56) Entgegenhaltungen:
- EP-A1- 0 493 819
- WO-A1-2006/106071
- DE-A1- 10 151 232

## Beschreibung

Die vorliegende Erfindung betrifft ein Transportbehältersystem für eine austauschbare Wasseranalyse-Sensorkartusche.

Wasseranalyse-Tauchsonden werden insbesondere in der Prozessanalytik zur Überwachung eines oder mehrerer Analyte in Wasser eingesetzt, um die Qualität von Wasser, beispielsweise Trinkwasser oder Abwasser, zu überwachen. Zur Vereinfachung der Wartung sind Tauchsonden nach dem Stand der Technik modular aufgebaut, und bestehen im Wesentlichen aus einer fest installierten Sondenbasis und einer austauschbaren Sensorkartusche mit einem oder mehreren unterschiedlichen Sensoren.

Da die Sensoren mit der Zeit Verschleiß unterliegen, durch den sich die Qualität der Messung zunehmend verschlechtert, ist es zweckmäßig, durch einen einfachen Austausch der Sensorkartusche neue und unverschlissene Sensoren wieder zur Verfügung zustellen.

Die Sensorkartusche ist relativ empfindlich und ihre Sensoren, insbesondere die Sensormembranen, dürfen je nach ihrer Beschaffenheit nicht austrocknen. Der Transport einer neuen unverbrauchten Sensorkartusche erfolgt daher in einem Transportbehälter, der eine spezifische Elektrolytlösung, d.h. ein Feuchthaltemittel, aufweist.

Sensorkartuschen können mit unterschiedlich selektiven Sensoren ausgestattet sein, d.h. mit Kationen- und/oder Anionen-selektiven Sensormembranen. Hierdurch kommt es bei der Lagerung derartiger Sensorkartuschen in einem Transportbehälter, der mit nur einer einzigen Elektrolytlösung befüllt ist, zu einer Diffusion der in den Sensormembranen vorhandenen messtechnisch aktiven Bestandteile, den sog. Ionophoren, in die Elektrolytlösung. Die Diffusion der Ionophore der jeweiligen Sensormembranen in die Elektrolytlösung führt zu einer Kontamination der jeweils anderen Sensormembranen der Sensorkartusche. Durch die Kontamination werden die Sensormembranen derart chemisch verändert, dass sie fehlerhaft auf die im Wasser vorliegenden Analyte reagieren.

Eine austauschbare Wasseranalyse-Sensorkartusche mit mehreren Sensormembranen ist bekannt aus WO 2006/106071 A1.

Aus EP 0493819 A1 ist eine Sensoranordnung bekannt, deren Sensoren mit einer Kalibrierkartusche kalibriert werden können, die eine Kalibrierflüssigkeit enthält.

Aus DE 101 51 232 A1 ist ein pH-Sensor bekannt, dessen Elektrode in einem Transportbehälter eingesteckt werden kann, der einen Schwamm mit einer Flüssigkeit 10 enthält.

Aufgabe der vorliegenden Erfindung ist es demgegenüber, ein Transportbehältersystem für eine austauschbare Wasseranalyse-Sensorkartusche zu schaffen, das eine Kontamination der Sensormembranen einer austauschbaren Sensorkartusche während des Transports in einem Transportbehältersystem verhindert.

Diese Aufgabe wird erfindungsgemäß gelöst durch ein Transportbehältersystem für eine austauschbare Wässeranalyse-Sensorkartusche mit den Merkmalen des Patentanspruchs 1.

Das Transportbehältersystem für eine austauschbare Wasseranalyse-Sensorkartusche besteht aus einer austauschbaren Wasseranalyse-Sensorkartusche, die mindestens zwei unterschiedliche Sensormembranen aufweist, und einem Transportbehälter-Becher, der für jede Sensormembran eine separate Feuchthaltekammer mit jeweils einer Klammeröffnung aufweist, wobei die Feuchthaltekammern ein für die Sensormembranen spezifisches Feuchthaltemittel aufweisen, Durch die Kammeröffnung wird das Feuchthaltemittel in die Feuchthaltekammer eingebracht. Ferner wird durch die Kammeröffnung der Kontakt zwischen den Sensormembranen und dem darin angeordneten Feuchthaltemittel ermöglicht.

Zum Transport wird die austauschbare Sensorkartusche in den Transportbehälter-Becher eingesetzt. Hierbei wird gewährleistet, dass die Sensormembranen separat und in einem für sie speziellen Feuchthaltemittel aufbewahrt werden können, so dass eine Kontamination der jeweils anderen Sensormembran ausgeschlossen ist. Speziell für die unterschiedlichen Sensormembranen verwendete Feuchthaltemittel gewährleisten eine optimale Leistung der Sensormembran sofort nach Entnahme, d.h. die Sensorkartusche ist sofort einsatzfähig.

Vorzugsweise sind die separaten Feuchthaltekammern durch jeweils eine im Wesentlichen zylindrische Kammerwand gebildet, die jeweils senkrecht auf einem Boden des Transportbehälter-Bechers steht. Alternativ ist jede andere zu den Sensormembranen komplementäre geometrische Form der Feuchthaltekammer denkbar.

Vorzugsweise ist mindestens ein spezifisches Feuchthaltemittel ein Elektrolytgel. Alternativ kann mindestens ein spezifisches Feuchthaltemittel durch einen Schwammkörper gebildet werden, weicher mit einer Elektrolytlösung getränkt ist. Ein derartiges spezifisches Feuchthaftemittel gewährleistet eine hundertprozentige Luftfeuchtigkeit an den Sensormembranen, die mit dem Feuchthaltemittel in direktem Kontakt stehen.

Gemäß einer bevorzugten Ausgestaltung ist ein flüssigkeitsdichter Transportbehälter-Deckel vorgesehen, der den Transportbehälter-Becher flüssigkeitsdicht abschließt. Hierdurch kann die austauschbare Sensorkartusche sicher transportiert und gelagert werden, ohne dass eine signifikante Verschlechterung der Sensormembranen auftritt. Zudem bietet der Transportbehälter-Deckel einen Schutz für die elektrischen Kontaktstellen der Sensorkartusche, über die die Sensorkartusche mit einer Sondenbasis einen elektrischen Kontakt bildet.

Vorzugsweise weist die Wasseranalyse-Sensorkartusche einen umlaufenden Flansch auf, der flüssigkeitsdicht an dem Transportbehälter-Becherrand anliegt, wobei ein Transportbehälter-Deckel zur Fixierung der Wasseranalyse-Sensorkartusche als Fixiermuffe ausgebildet ist. Durch diesen Anschlag kann die Sensorkartusche optimal in dem Trarisportbehälter-Becher gelagert werden, d.h. der Abstand zwischen den Sensormembranen und den korrespondierenden Feuchthaltekammern kann derart und definiert eingestellt werden, dass die Sensormembran jeweils optimal an dem Feuchthaltemittel anliegt. Ferner bildet diese umlaufende Flanschfläche auch eine Anschlagfläche für die Verbindung der Sensorkartusche mit einer Sondenbasis.

Gemäß einer bevorzugten Ausgestaltung sind der Transportbehälter-Becher und der Transportbehälter-Deckel mit einem zusammenwirkenden Bajonettverschluss versehen. Ein Bajonettverschluss ist eine schnell verschließbare, zuverlässig haltende und leicht lösbare mechanische Verbindung.

Vorzugsweise weist der Transportbehälter-Deckel außenseitig eine über den Umfang angeordnete Greifstruktur auf, die das Ergreifen des Deckels vereinfacht und insbesondere in Umfangsrichtung zum Drehen des Deckels einen höheren Kraftschluss zwischen der ergreifenden Hand und dem Transportbehälter-Deckel ermöglicht. Zudem kann auch der Transportbehälter-Becher diese Greifstruktur aufweisen, um das Öffnen des Transportbehältersystems insgesamt zu vereinfachen.

Vorzugsweise ist mindestens eine der Sensormembranen der im Wesentlichen zylindrisch ausgebildeten Wasseranalyse-Sensorkartusche exzentrisch darin angeordnet. Hierdurch wird eine maximale Raumausnutzung der Sensorkartusche für die Sensormembranen realisiert, so dass eine Vielzahl unterschiedlicher Sensoren in einer einzigen Sensorkartusche vorgesehen werden können. Analog hierzu weist der Transportbehälter-Becher die gleiche Anzahl an Feuchthaltekammern auf, so dass jede der Sensormembranen separat und in einem für sie speziellen Feuchthaltemittel gelagert werden kann.

Gemäß einer bevorzugten Ausgestaltung weisen die Wasseranalyse-Sensorkartusche und der Transportbehälter-Becher zusammenwirkende Verdrehsicherungen auf, die beispielsweise als senkrecht auf dem Transportbehälter-Becherrand stehende Zapfen realisiert sind und die mit entsprechenden Öffnungen in dem umlaufenden Flansch der Sensorkartusche korrespondieren. Dadurch wird sichergestellt, dass die Sensormembranen der Sensorkartusche in den für sie vorgesehenen Feuchthaltekammern während des Transports und/oder der Lagerung angeordnet sind.

In einer besonders bevorzugten Ausgestaltung weist der Transportbehälter-Becher ein Absorptionsmittel im Becher-Innenraum auf. Das Absorptionsmittel ist stets in Bezug auf den Becher-Innenraum wirksam, auch bei in den Transportbehälter-Becher eingesetzter Sensorkartusche. Vorzugsweise ist das Absorptionsmittel ein Schwammkörper. Alternativ kann das Absorptionsmittel aus einem hygroskopischen Mittel, wie beispielsweise Kieselgel, bestehen. Das Absorptionsmittel kann beispielsweise in einer dafür vorgesehenen Kammer, welche mittig auf dem Boden des Transportbehälter-Bechers angeordnet ist, untergebracht sein. Das Absorptionsmittel kann beispielsweise überschüssige Elektrolytlösung aufnehmen, wenn diese aus den Feuchthaltekammern austritt, so dass eine Vermischung der unterschiedlichen Elektrolytlösungen untereinander wirksam verhindert wird.

Nachfolgend wird ein Ausführungsbeispiel der Erfindung anhand der Zeichnung näher erläutert.
Figur 1 zeigt eine Explosionsdarstellung des erfindungsgemäßen Transportbehältersystems.
Figur 2 zeigt eine Schnittdarstellung des erfindungsgemäßen Transportbehältersystems.

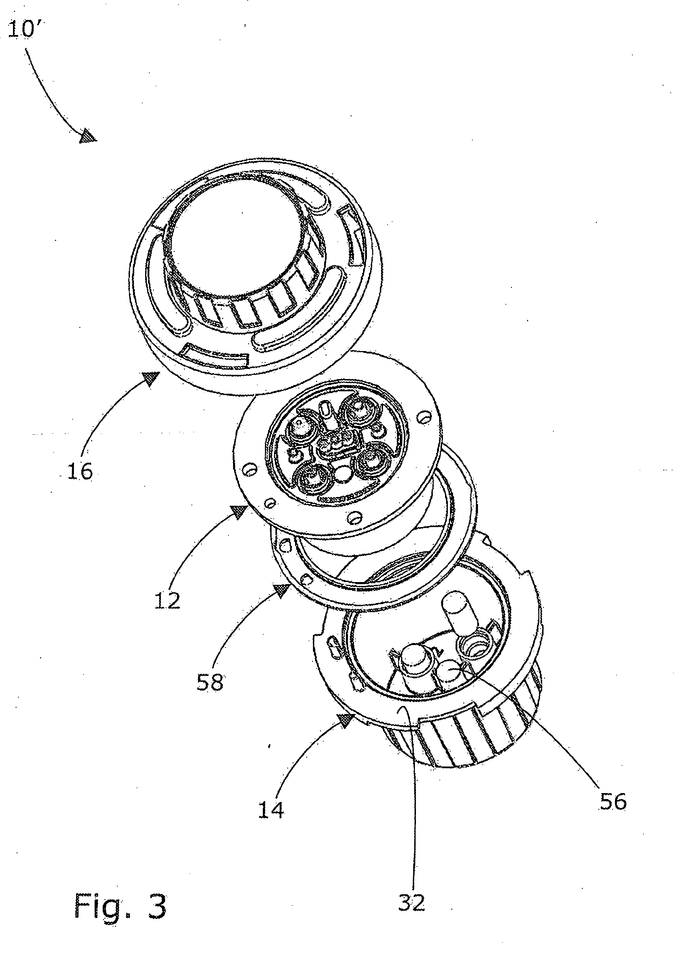

Figur 3 zeigt eine Explosionsdarstellung einer alternativen Ausführungsform des erfindungsgemäßen Transportbehältersystems.

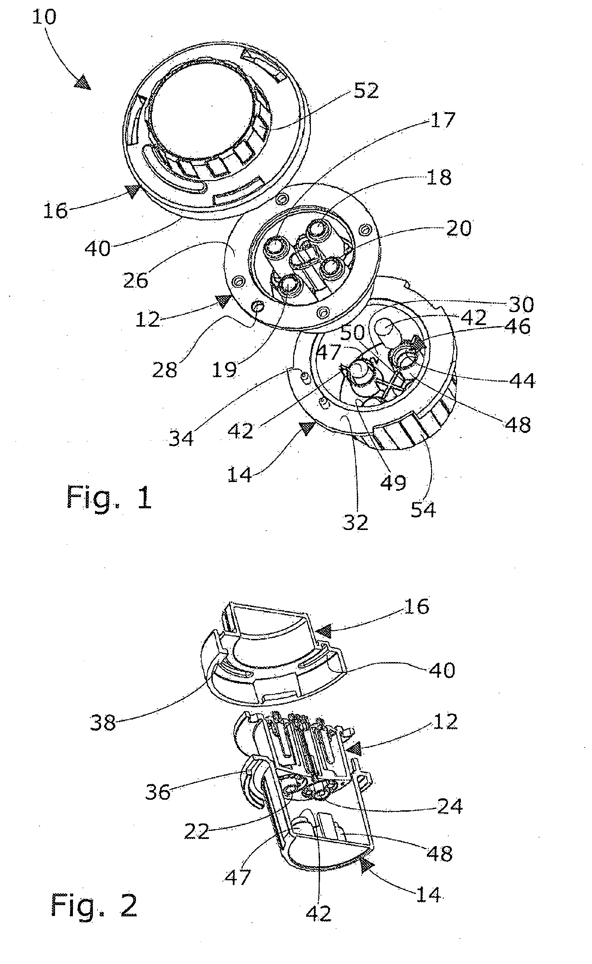

In Figur 1 ist ein Transportbehältersystem 10 für eine austauschbare Wasseranalyse-Sensorkartusche 12 gezeigt. Das System 10 besteht aus einer austauschbaren Sensorkartusche 12, die zusammen mit einer Sondenbasis (nicht dargestellt) eine Wasseranalyse-Tauchsonde bildet, und einem für die Sensorkartusche 12 vorgesehenen Transportbehälter-Becher 14, wobei der Transportbehälter-Becher 14 mit einem Transportbehälter-Deckel 16 verschließbar ist.

Die Sensorkartusche 12 weist vier unterschiedliche Sensoren 17, 18, 19, 20 auf. Die vier Sensoren 17, 18, 19, 20 weisen an ihren distalen Enden Sensormembranen 22, 24 (siehe Figur 2) auf. Die Sensoren 17, 18, 19, 20 sind in der im Wesentlichen zylindrisch ausgebildeten Sensorkartusche 12 exzentrisch angeordnet, so dass eine optimale Raumausnutzung der Sensorkartusche 12 ermöglicht wird. Ferner weist die Sensorkartusche 12 einen umlaufenden Flansch 26 mit darin vorgesehenen runden Öffnungen 28 auf.

Der im Wesentlichen zylindrische Transportbehälter-Becher 14 besteht aus Kunststoff und ist an seinem Boden 30 geschlossen. Der Becher 14 weist an dem anderen Längsende eine Öffnung mit einem flanschartigen Becherrand 32 auf. Auf dem Becherrand 32 sind axial stehende Zapfen 34 vorgesehen, die zusammen mit entsprechenden Öffnungen 28 im Flansch 26 der Sensorkartusche 12 korrespondieren und zusammen eine Verdrehsicherung bilden. Ferner weist der Becher 14 an seinem Becherrand 32 mehrere Bajonett-Elemente 36 (siehe Figur 2) auf, die mit entsprechenden Bajonett-Nasen 38 (Figur 2) des Deckels 16 einen Bajonettverschluss bilden, so dass dieser mit seinem flanschartigen Deckelrand 40 auf dem Flansch 26 der Sensorkartusche 12 verdreht wird.

Im Inneren weist der Becher 14 für jede der Sensormembranen 22, 24 der Sensorkartusche 12 separate Feuchthaltekammern 47, 48, 49, 50 auf, die jeweils ein für die Sensormembran 22, 24 bzw, für die Sensoren 17, 18, 19, 20 spezifisches Feuchthaltemittel 42 enthalten. Das Feuchthaltemittel 42 ist beispielsweise ein Schwammkörper, der mit einer Elektrolytlösung getränkt ist oder ein Elektrolytgel. Ferner weist der Becher 14 im Inneren ein Feuchtigkeits-Absorptionsmittel 56 auf, welches beispielsweise durch einen Schwammkörper gebildet wird oder aus einem hygroskopischen Mittel besteht.

Die Feuchthaltekammern 47 - 50 werden durch im Wesentlichen zylindrische ausgebildete Kammerwände 44 gebildet, die senkrecht auf dem Boden 30 des Bechers 14 stehen. Die Kammerwände 44 weisen nur an der zum Boden 30 entgegengesetzten Seite eine Kammeröffnung 46 auf, in die das Feuchthaltemittel 42, beispielsweise in Form eines Schwammkörpers oder eines Elektrolytgels (nicht dargestellt), eingesetzt ist.

Außenseitig weist der Transportbehälter-Deckel 16 eine über den Umfang angeordnete Greifstruktur 52 auf, die das Ergreifen des Deckels 16 vereinfacht. Auch der Transportbehälter-Becher 14 weist eine außenseitig liegende Greifstruktur 54 auf, die das Öffnen des Transportbehältersystems, 10 insgesamt vereinfacht

Im Transport- bzw. Lagerzustand ist die Sensorkartusche 12 mit dem Flansch 26 axial zwischen dem flanschartigen Deckelrand 40 des Deckels 16 und dem flanschartigen Becherrand 32 des Bechers 14 fixiert, so dass die Sensorkartusche 12 optimal in dem Transportbehälter-Becher 14 gelagert werden kann, d.h. der Abstand zwischen den Sensormembranen 22, 24 und den korrespondierenden Feuchthaltekammern 47, 48 (Figur 2) kann derart und definiert eingestellt werden, dass die Sensormembran 22, 24 jeweils optimal an dem Feuchthaltemittel 42 anliegt. Alternativ kann zwischen der Sensorkartusche 12 und dem flanschartigen Becherrand 32 des Bechers 14 ein Dichtring 58 vorgesehen sein, so dass das Transportbehältersystem 10' flüssigkeitsdicht, zuverlässig verschlossen werden kann.

## Patentansprüche

1. Transportbehältersystem (10, 10') für eine austauschbare Wasseranalyse-Sensorkartusche, mit
einer austauschbaren Wasseranalyse-Sensorkartusche (12), die mindestens zwei unterschiedliche Sensormembranen (22, 24) aufweist, und
einem Transportbehälter-Becher (14), der für jede Sensormembran (22, 24) eine separate Feuchthaltekammer (47, 48) mit jeweils einer Kammeröffnung (46) aufweist, wobei die Feuchthaltekammern (47, 48) ein für die Sensormembranen (22, 24) spezifisches Feuchthaltemittel (42) aufweisen.

2. Transportbehältersystem (10, 10') nach Anspruch 1, wobei die separaten Feuchthaltekammern (47, 48) durch jeweils eine im Wesentlichen zylindrische Kammerwand (44) gebildet werden, die senkrecht auf einem Boden (30) des Transportbehälter-Bechers (14) stehen.

3. Transportbehältersystem (10, 10') nach Anspruch 1 oder 2, wobei mindestens ein spezifisches Feuchthaltemittel (42) ein Elektrolytgel ist.

4. Transportbehältersystem (10, 10') nach einem der Ansprüche 1 - 2, wobei mindestens ein spezifisches Feuchthaltemittel (42) durch einen Schwammkörper gebildet wird, welcher mit einer Elektrolytlösung getränkt ist.

5. Transportbehältersystem (10, 10') nach einem der vorangegangenen Ansprüche, wobei ein flüssigkeitsdichter Transportbehälter-Deckel (16) vorgesehen ist, der den Transportbehälter-Becher (14) flüssigkeitsdicht abschießt:

6. Transportbehältersystem (10, 10') nach einem der vorangegangenen Ansprüche, wobei die Wasseranatyse-Sensorkartusche (12) mit einem umlaufenden Flansch (26) flüssigkeitsdicht an dem Transportbehälter-Becherrand (32) anliegt, wobei ein Transportbehälter-Deckel (16) zur Fixierung der Wasseranalyse-Sensorkartusche (12) als Fixiermuffe ausgebildet ist.

7. Transportbehältersystem (10, 10') nach einem der Ansprüche 5 und 6, wobei der Transportbehälter-Becher (14) und der Transportbehälter-Deckel (16) mit einem zusammenwirkenden Bajonettverschluss versehen sind.

8. Transportbehältersystem (10, 10') nach einem der Ansprüche 5 - 7, wobei der Transportbehälter-Deckel (16) außenseitig eine über den Umfang angeordnete Greifstruktur (52) aufweist.

9. Transportbehältersystem (10, 10') nach einem der vorangegangenen Ansprüche, wobei der Transportbehälter-Becher (14) außenseitig eine über den Umfang angeordnete Greifstruktur (54) aufweist.

10. Transportbehältersystem (10, 10') nach einem der vorangegangenen Ansprüche, wobei mindestens eine der Sensormembranen (22, 24) der im Wesentlichen zylindrisch ausgebildeten Wasseranalyse-Sensorkartusche (12) exzentrisch darin angeordnet ist.

11. Transportbehältersystem (10, 10') nach einem der vorangegangenen Ansprüche, wobei Wasseranalyse-Sensorkartusche (12) und der Transportbehälter-Becher (14) zusammenwirkende Verdrehsicherungen aufweisen.

12. Transportbehältersystem (10, 10') nach einem der vorangegangenen Ansprüche, wobei der Transportbehälter-Becher (14) im Innern ein Absorptionsmittel (56) aufweist.

13. Transportbehältersystem (10, 10') nach Anspruch 12, wobei das Absorptionsmittel (56) durch einen Schwammkörper gebildet wird.

14. Transportbehältersystem (10, 10') nach Anspruch 12, wobei das Absorptionsmittel (56) aus einem hygroskopischen Mittel besteht.

## Claims

1. A transport container system (10, 10') for an interchangeable water analysis sensor cartridge, comprising:
an Interchangeable water analysis sensor cartridge (12) comprising at least two different sensor membranes (22, 24), and
a transport container cup (14) comprising, for each of the at least two different sensor membranes (22, 24), a separate moist chamber (47, 48), each comprising one chamber opening (46), the moist chambers (47, 48) comprising a specific humectant (42) for the sensor membranes (22, 24).

2. The transport container system (10, 10') as recited in claim 1, wherein each separate moist chamber (47, 48) is formed by a respective cylindrical chamber wall (44) arranged in the vertical orientation on the bottom (30) of the transport-container cup (14).

3. The transport container system (10, 10') as recited in claim 1 or 2, wherein at least one specific humectant (42) Is an electrolyte gel.

4. The transport container system (10, 10') as recited in one of claims 1 - 2, wherein at least one specific humectant (42) is formed by a sponge body soaked with an electrolyte solution.

5. The transport container system (10, 10') as recited in one of the preceding claims, wherein a liquid-tight transport container lid (16) is provided that is configured to provide for a liquid-tight closure of the transport container cup (14).

6. The transport container system (10, 10') as recited In one of the preceding claims, wherein the water analysis sensor cartridge (12) has a surrounding flange (20) in a liquid-tight abutment on the transport container cup edge (32), the transport container lid (16) being formed as a fixing sleeve for fixing the water analysis sensor cartridge (12).

7. The transport container system (10, 10') as recited in one of claims 5 and 6, wherein the transport container cup (14) and the transport container lid (16) are provided with a cooperating bayonet-type lock.

8. The transport container system (10, 10') as recited in one of claims 5 - 7, wherein the transport container lid (16) has a gripping structure (52) arranged on the outer side along the periphery.

9. The transport container system (10, 10') as recited in one of the preceding claims, wherein the transport container cup (14) has a gripping structure (54) arranged on the outer side along the periphery.

10. The transport container system (10, 10') as recited in one of the preceding claims, wherein at least one of the at least two sensor membranes (22, 24) is arranged eccentrically in the substantially cylindrical water analysis sensor cartridge (12).

11. The transport container system (10, 10') as recited In one of the preceding claims, wherein the water analysis sensor cartridge (12) and the transport container cup (14) are provided with cooperating anti-twist locks.

12. The transport container system (10, 10') as recited in one of the preceding claims, wherein the transport container cup (14) comprises an absorption agent (56) arranged in the interior.

13. The transport container system (10, 10') as recited in claim 12, wherein the absorption agent (56) Is formed by a sponge body.

14. The transport container system (10, 10') as recited in claim 12, wherein the absorption agent (56) consists of a hygroscopic medium.

## Revendications

1. Système de conteneur de transport (10, 10') pour une cartouche de capteur d'analyse de l'eau remplaçable, comprenant
une cartouche de capteur d'analyse de l'eau (12) remplaçable, ayant au moins deux membranes de capteur (22, 24) différentes, et
une cuvette de conteneur de transport (14) comprenant une chambre d'humidification (47, 48) séparée pour chaque membrane de capteur (22, 24), chaque chambre ayant une ouverture de chambre (46), lesdites chambres d'humidification (47, 48) contenant un humectant (42) spécifique des membranes de capteur (22, 24).

2. Système de conteneur de transport (10, 10') selon la revendication 1, dans lequel lesdites chambres d'humidification (47, 48) sont formées, respectivement, par une paroi (44) essentiellement cylindrique posée verticalement sur un fond (30) de ladite cuvette de conteneur de transport (14).

3. Système de conteneur de transport (10, 10') selon la revendication 1 ou 2, dans lequel au moins un humectant (42) spécifique est un gel électrolytique.

4. Système de conteneur de transport (10, 10') selon l'une quelconque des revendications 1 - 2, dans lequel au moins un humectant (42) spécifique est formé par un corps spongieux imprégné avec une solution électrolytique.

5. Système de conteneur de transport (10, 10') selon l'une quelconque des revendications précédentes, dans lequel un couvercle de conteneur de transport (16) étanche à liquide est prévu, ledit couvercle fermant ladite cuvette du conteneur de transport (14) de manière étanche à fluide.

6. Système de conteneur de transport (10, 10') selon l'une quelconque des revendications précédentes, dans lequel ladite cartouche de capteur d'analyse de l'eau (12), par une bride (26) périphérique, est en contact étanche à liquide avec le bord (32) de la cuvette de conteneur de transport, un couvercle de conteneur de transport (16) étant réalisé en forme d'un manchon de fixation pour fixer ladite cartouche de capteur d'analyse de l'eau (12).

7. Système de conteneur de transport (10, 10') selon l'une quelconque des revendications 5 et 6, dans lequel ladite cuvette du conteneur de transport (14) et ledit couvercle de conteneur de transport (16) sont munis d'une fermeture à baïonnette complémentaire.

8. Système de conteneur de transport (10, 10') selon l'une quelconque des revendications 5 - 7, dans lequel ledit couvercle de conteneur de transport (16) comprend, sur le coté extérieur, une structure de prise (52) disposée suivant la périphérie.

9. Système de conteneur de transport (10, 10') selon l'une quelconque des revendications précédentes, dans lequel ladite cuvette du conteneur de transport (14) comprend, sur le coté extérieur, une structure de prise (52) disposée suivant la périphérie.

10. Système de conteneur de transport (10, 10') selon l'une quelconque des revendications précédentes, dans lequel au moins une des membranes de capteur (22, 24) de ladite cartouche de capteur d'analyse de l'eau (12) est disposée dans celle-ci de manière excentrique, la cartouche étant sensiblement cylindrique.

11. Système de conteneur de transport (10, 10') selon l'une quelconque des revendications précédentes, dans lequel ladite cartouche de capteur d'analyse de l'eau (12) et ladite cuvette du conteneur de transport (14) comprennent des moyens anti-rotation complémentaires.

12. Système de conteneur de transport (10, 10') selon l'une quelconque des revendications précédentes, dans lequel ladite cuvette du conteneur de transport (14) contient un agent d'absorption (56) à l'intérieur.

13. Système de conteneur de transport (10, 10') selon la revendication 12, dans lequel ledit agent d'absorption (56) est un corps spongieux.

14. Système de conteneur de transport (10, 10') selon la revendication 12, dans lequel ledit agent d'absorption (56) est un agent hygroscopique.
